# EUROPEAN PATENT SPECIFICATION

(11) **EP 2 499 972 B1**
(45) Date of publication and mention of the grant of the patent: **11.10.2017**
(21) Application number: 10829907.4
(22) Date of filing: 08.11.2010
(51) Int. Cl.: A61B 6/03, G06T 7/00, A61B 6/00

(54) **MEDICAL THREE-DIMENSIONAL IMAGE DISPLAY-ORIENTATION ADJUSTMENT DEVICE AND ADJUSTMENT PROGRAM**
VORRICHTUNG UND PROGRAMM ZUR EINSTELLUNG DER AUSRICHTUNG DREIDIMENSIONALER BILDANZEIGEN
DISPOSITIF PERMETTANT DE RÉGLER L'ORIENTATION D'AFFICHAGE D'UNE IMAGE MÉDICALE TRIDIMENSIONNELLE ET PROGRAMME DE RÉGLAGE

(30) Priority: 13.11.2009 JP 2009260077; 26.11.2009 JP 2009268620
(43) Date of publication of application: 19.09.2012
(73) Proprietor: Imagnosis Inc., Kobe-shi, Hyogo 658-0032 (JP)
(72) Inventor: KIM, Han-Joon, Kobe-shi Hyogo 658-0032 (JP)
(74) Representative: Witte, Weller & Partner Patentanwälte mbB
(86) International application number: PCT/JP2010/069837
(87) International publication number: WO 2011/058948

(56) References cited:
- EP-A1- 1 207 495
- EP-A1- 1 992 287
- WO-A1-2008/047270
- WO-A1-2008/120136
- JP-A- 2002 360 564
- JP-A- 2003 265 475
- US-A1- 2008 300 477

## Description

### Technical Field

The present invention relates to a device and a program for adjusting a display orientation of a medical three-dimensional image.

### Background Art

When treating jaw deformity involving jaw distortion in the dental or plastic surgery field, for grasping functional and aesthetic maxillofacial problems, diagnosis of the maxillofacial morphology is important. Conventionally, a cephalometric radiograph is key information for diagnosis of maxillofacial morphology, however, image magnification and distortion occurs in a radiograph. In a radiograph of the side of a face, by taking a midpoint of a bilaterally symmetric structure, compensation can be made for a difference in magnification factor between the left and right structure projection images, so that a big problem does not occur. However, in a radiograph of a frontal face, an X-ray is irradiated in the front-back direction, so that compensation cannot be made for a difference in magnification factor between the left and right structures, and for each time of photographing, due to a head position error, the magnification factor becomes different between the left and right in a radiographic image, so that it is difficult to evaluate the bilateral symmetry of the face.

In the cephalometric radiography, a cephalometric radiograph is taken in a state where rods called ear rods are fitted to the left and right ear canals, however, the left and right ears are not always bilaterally symmetrical, so that the left and right ears are not absolute elements for determining the frontal face orientation. However, in the conventional radiography, there is no means for positioning the head position other than ear rods, so that only the frontal face orientation based on the ear rods can be standardized.

### Prior Art Documents

### Patent Documents

Patent Document 1: Japanese Published Unexamined Patent Application No. H08-131403
Patent Document 2: Japanese Published Unexamined Patent Application No. 2002-360564
Patent Document 3: Japanese Published Unexamined Patent Application No. 2007-130240

### Summary Of the Invention

### Problems to be Solved by the Invention

In recent years, it has been tried to apply a CT (Computed Tomography) image three-dimensionally constructed based on three-dimensional CT data to frontal face evaluation. However, the CT is not standardized photography, and when evaluating the bilateral symmetry of a face by using a CT image, first, instead of using the coordinate system with low clinical reproducibility and low validity of the CT image adopted when photographing as it is, the coordinate system must be re-positioned in a frontal orientation preferable for morphological evaluation. As a positioning means for re-positioning the coordinate system, a positioning means that can reflect a large number of anatomical maxillofacial feature points (hereinafter, referred to as "landmarks") and can set various frontal orientations is required. The inventor of the present invention has already developed a method for setting a frontal face orientation by setting an anatomical or morphological reference surface based on landmarks on a CT image and a method for correcting a set frontal face orientation by further using desired landmarks as reference points, and applied for a patent (refer to Patent Documents 2 and 3 listed above). These methods enable setting of various orientations with high reproducibility and high validity required for utilizing CT images for morphological diagnosis.

However, many landmarks are defined for morphological measurement on a radiophotograph, so that when three-dimensionally identifying the two-dimensionally defined landmarks, an error occurs, and for example, it is difficult to reflect a region such as the buccal region of a face in which landmarks cannot be set morphologically due to its shape in positioning. In addition, the length of a side of a minimum constituent unit (voxel) of CT data is approximately 0.5 mm at minimum, so that it is impossible to specify landmarks with high accuracy on the CT data. Therefore, a positioning method that does not depend on landmark specification and a method for correcting a positioning error caused by landmark specification have been demanded.

Further, for a patient who was positioned in an orientation for evaluation of a CT image in the past, CT is newly performed, and to quantitatively evaluate a change between a past CT and a new CT, a method for reproducing the past positioning orientation with high accuracy is demanded. In addition, when an orientation of a face that a patient recognizes or desires on his/her own is photographed with a camera or a three-dimensional camera, and when evaluating the symmetry of the maxillofacial skeleton by using a CT image, a method for matching the orientation of the CT image and the orientation of the photograph taken with the camera with high accuracy is demanded. Thus, as CT images are increasingly utilized for morphological diagnosis, setting of greater variation in orientation is demanded.

An object of the present invention is to further improve diagnostic and treatment correctness by further improving variation, accuracy, and operability of positioning of a medical three-dimensional image.

Document WO2008047270 A1 may be considered to disclose a display orientation adjustment device for a medical three dimensional image, comprising: a means for making a display means display a medical three-dimensional image based on medical three-dimensional image data according to a predetermined three-dimensional reference coordinate system; a means for making the display means display a reference image; an observation direction change command input means for inputting an observation direction change command for changing an observation direction of a display image displayed by the display means; an observation direction changing means for moving the whole display image including the medical three-dimensional image and the reference image according to an observation direction change command input by the observation direction change command input means; as well as the corresponding computer readable medium.

### Means for Solving the Problems

A first aspect of the present invention provides a display orientation adjustment device for a medical three-dimensional image, including a means (1) for making a display means (2) display a medical three-dimensional image (11) based on medical three-dimensional image data according to a predetermined three-dimensional reference coordinate system (Xo, Yo, Zo), a means (1) for making the display means display a reference image (12) to be used as a reference for adjustment of a display orientation of the medical three-dimensional image, an observation direction change command input means (4, 50) for inputting an observation direction change command for changing an observation direction of a display image displayed by the displaymeans, an observation direction changing means (1) for moving the whole display image including the medical three-dimensional image and the reference image according to an observation direction change command input by the observation direction change command input means, a display orientation change command input means (4, 60) for inputting a command for rotatively moving only the medical three-dimensional image of the medical three-dimensional image and the reference image displayed by the display means around a first image reference axis specified among image reference axes of the three-dimensional reference coordinate system, a display orientation changing means (1) for rotatively moving only the medical three-dimensional image around the first image reference axis according to a display orientation change command input by the display orientation change command input means, and a first correcting means (1) for correcting medical three-dimensional image data on the three-dimensional reference coordinate system according to the rotative movement of the medical three-dimensional image by the display orientation changing means. The alphanumeric characters in parentheses indicate corresponding constituent elements, etc. , in the preferred embodiments described later. The same applies hereinafter in this section.

In the first aspect of the present invention, a medical three-dimensional image is displayed by the display means based on medical three-dimensional image data according to a predetermined three-dimensional reference coordinate system, and a reference image to be used as a reference for adjustment of the display orientation of the medical three-dimensional image is displayed by the display means. As the reference image, for example, a plane, cells, or a face symmetric model outline orthogonal to an image reference axis (hereinafter, referred to as "third image reference axis") set or specified for displaying the reference image, contour lines or a moire projected in the third image reference axis direction, etc., can be used.

On a plane orthogonal to the third image reference axis, a photograph of a patient's face or a face feature outline extracted from the photograph of the patient's face may be displayed as a reference image. Alternatively, an image that was photographed and positioned in a display orientation in the past and is in an orientation orthogonal to the third image reference axis with respect to amedical three-dimensional image newly photographed may be displayed on the plane orthogonal to the image reference axis. Instead of displaying the reference image on a plane orthogonal to the third image reference axis, a reference image (for example, photograph, two-dimensional image, or outline, etc.) oriented corresponding to an observation direction of the medical three-dimensional image is displayed on a plane orthogonal to the observation direction (viewpoint direction).

When an observation direction change command is input, according to the input observation direction change command, the whole display image including the medical three-dimensional image and the reference image is moved. When a display orientation change command is input, according to the input display orientation change command, only the medical three-dimensional image is rotatively moved around a specified image reference axis (first image reference axis) by the display orientation changing means. According to the rotative movement of the medical three-dimensional image by the display orientation changing means, medical three-dimensional image data on the three-dimensional reference coordinate system is corrected.

According to the present invention, a reference image to be used as a reference for adjustment of the display orientation of a medical three-dimensional image is displayed, so that the display orientation of the medical three-dimensional image is easily adjusted. For example, when adjusting the frontal face orientation of a head image, by displaying a plane, cells, or a face symmetric model outline orthogonal to an image reference axis (Yo axis) extending in the front-back direction of the face, contour lines or a moire projected in the image reference axis direction, etc. , as a reference image, the frontal face orientation is easily adjusted based on the bilateral symmetry of the face.

In addition, according to the present invention, the display orientation of the medical three-dimensional image can be adjusted from a plurality of observation directions, so that the display orientation can be adjusted more accurately. Further, according to the present invention, according to rotative movement of the medical three-dimensional image by the display orientation changing means, the medical three-dimensional image data according to the three-dimensional reference coordinate system is corrected, so that it is not necessary to specify landmarks that are hard to accurately specify.

A second aspect of the present invention provides the display orientation adjustment device for a medical three-dimensional image according to the first aspect, wherein the first correcting means corrects two image reference axes other than the specified one image reference axis based on the first image reference axis and a direction and a magnitude of rotative movement of the medical three-dimensional image by the display orientation changing means. In the second aspect of the present invention, based on the first image reference axis and a direction and a magnitude of rotative movement of the medical three-dimensional image by the display orientation changingmeans, two image reference axes other than the specified one image reference axis are corrected.

A third aspect of the present invention provides the display orientation adjustment device for a medical three-dimensional image according to the first or second aspect, further including a parallel movement command input means (4, 60) for inputting a parallel movement command for parallel-moving the medical three-dimensional image displayed by the display means relative to a second image reference axis specified among the three-dimensional image reference axes, a parallel moving means (1) for parallel-moving the medical three-dimensional image displayed by the display means relative to the second image reference axis according to a parallel movement command input by the parallel movement command input means, and a second correcting means (1) for correcting medical three-dimensional image data on the three-dimensional reference coordinate system according to parallel movement by the parallel moving means.

In the third aspect of the present invention, when a parallel movement command is input, the medical three-dimensional image displayed by the display means is parallel-moved relative to the specified image reference axis (second image reference axis) by the parallel moving means according to the input parallel movement command. Then, according to parallel movement by the parallel moving means, medical three-dimensional image data on the three-dimensional reference coordinate system is corrected. For example, when the second image reference axis is the Yo axis, an origin that the Yo axis passes through can be moved relative to the medical three-dimensional image on the Xo-Zo plane. Similarly, when the second image reference axis is the Zo axis, an origin that the Zo axis passes through can be moved relative to the medical three-dimensional image on the Xo-Yo plane, and when the second image reference axis is the Xo axis, an origin that the Xo axis passes through can be moved relative to the medical three-dimensional image on the Yo-Zo plane. According to the present invention, by parallel-moving the medical three-dimensional image with respect to a specified image reference axis, the display orientation can be adjusted.

A fourth aspect of the present invention provides a display orientation adjustment program for a medical three-dimensional image, for making a computer (1) function as a means (1) for making a display means (2) display a medical three-dimensional image (11) based on medical three-dimensional image data according to a predetermined three-dimensional reference coordinate system (Xo, Yo, Zo), a means for making the display means display a reference image (12) to be used as a reference for adjustment of a display orientation of the medical three-dimensional image, an observation direction changing means for moving the whole display image including the medical three-dimensional image and the reference image according to a given observation direction change command, a display orientation changing means for rotatively moving only the medical three-dimensional image of the medical three-dimensional image and the reference image displayed by the display means around a first image reference axis specified among image reference axes of the three-dimensional reference coordinate system according to a given display orientation change command, and a first correcting means for correcting medical three-dimensional image data on the three-dimensional reference coordinate system according to the rotative movement of the medical three-dimensional image by the display orientation changing means. In the fourth aspect of the present invention, the same operation and effect as in the first aspect of the present invention can also be obtained.

A fifth aspect of the present invention provides the display orientation adjustment program for a medical three-dimensional image according to the fourth aspect, wherein the first correcting means corrects two image reference axes other than the first image reference axis based on the first image reference axis and a direction and a magnitude of the rotative movement of the medical three-dimensional image by the display orientation changing means. In the fifth aspect of the present invention, the same operation and effect as in the second aspect of the present invention can be obtained.

A sixth aspect of the present invention provides the display orientation adjustment program for a medical three-dimensional image according to the fourth or fifth aspect, further including a program for making a computer function as a parallel moving means for parallel-moving the medical three-dimensional image displayed by the display means relative to a second image reference axis specified among the three-dimensional image reference axes according to a given parallel movement command, and a means for correcting medical three-dimensional image data on the three-dimensional reference coordinate system according to parallel movement by the parallel moving means. The same operation and effect as in the third aspect of the present invention can also be obtained in the sixth aspect of the present invention.

### Effect of the Invention

According to the present invention, the display orientation of a medical three-dimensional image can be adjusted.

### BRIEF DESCRIPTION OF THE DRAWINGS

Fig. 1 is a block diagram showing a configuration of an image processing device according to a preferred embodiment of the present invention.
Fig. 2 is a schematic view showing an example of a medical three-dimensional image to be displayed on a display.
Fig. 3 is a flowchart showing steps of processing for adjusting the frontal face orientation.
Fig. 4 is a schematic view for describing parallel movement processing based on a frontal orientation adjusting operation by parallel movement.
Fig. 5 is a schematic view for describing rotative movement processing based on a frontal orientation adjusting operation by rotative movement.
Fig. 6 is a schematic view for describing rotative movement processing based on an observation direction changing operation and rotative movement processing based on a frontal orientation adjusting operation by rotative movement.
Fig. 7 is a schematic view for describing rotative movement processing based on a frontal orientation adjusting operation by rotative movement shown in Fig. 6.
Fig. 8 is a schematic view showing another example of rotative movement processing based on a frontal orientation adjusting operation by rotative movement.
Fig. 9 is a schematic view showing other examples of rotative movement processing based on an observation direction changing operation and rotative movement processing based on a frontal orientation adjusting operation by rotative movement.
Fig. 10 is a schematic view for describing rotative movement processing based on a frontal orientation adjusting operation by rotative movement shown in Fig. 9.

### Best Mode for Carrying Out the Invention

Hereinafter, preferred embodiments of the present invention are described with reference to the drawings. Preferred Embodiments

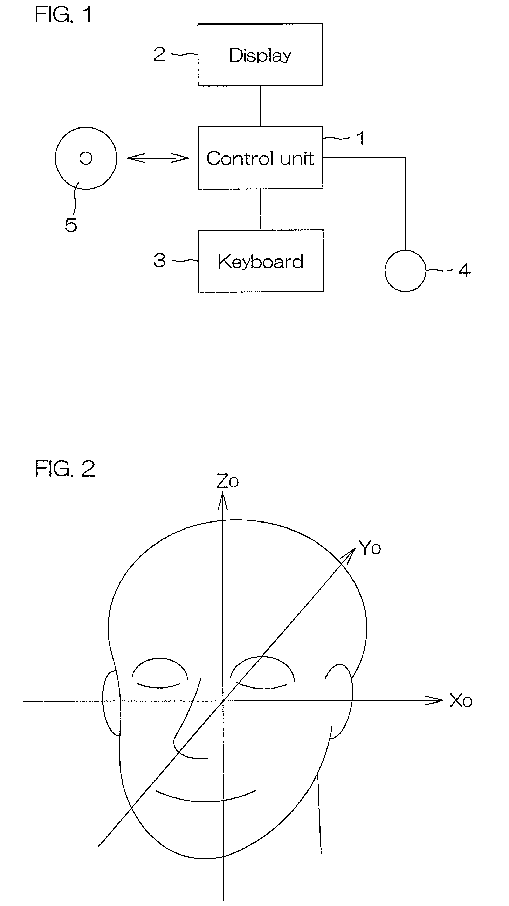

Fig. 1 shows a configuration of an image processing device according to a preferred embodiment of the present invention.

The image processing device is realized by, for example, a computer such as a personal computer (PC) . The image processing device includes a control unit 1 equipped with a CPU, a ROM, a RAM, and a hard disk, etc. To the control unit 1, a display (monitor) 2, a keyboard 3, a mouse 4, etc., are connected. On the hard disk of the control unit 1, a display orientation adjustment program according to a preferred embodiment of the present invention is installed from, for example, a storage medium 5, etc., storing the display orientation adjustment program.

The hard disk stores medical three-dimensional image data according to a predetermined three-dimensional reference coordinate system (body coordinate system: coordinate system having a reference point set at any point of a three-dimensional figure and using the reference point as an origin). In the present preferred embodiment, medical three-dimensional image data according to a predetermined three-dimensional reference coordinate system is three-dimensional image data created from CT (Computed Tomography) data and according to a three-dimensional reference coordinate system defined by using anatomical feature points (landmarks) in an image or three-dimensional image data corrected by the display orientation adjustment processing described later. Coordinate axes of the three-dimensional reference coordinate system are referred to as reference axes (image reference axes), and indicated as Xo, Yo, and Zo.

Medical three-dimensional image data according to a predetermined three-dimensional reference coordinate system may be data using a coordinate system that three-dimensional CT data has (coordinate system constructed when photographing) as the three-dimensional reference coordinate system, or may be data using a coordinate system obtained by correcting data on the coordinate system constructed when photographing by using landmarks and a reference surface, etc., as the three-dimensional reference coordinate system. The medical three-dimensional image data according to a predetermined three-dimensional reference coordinate systemmay be data using a coordinate system that the CT data read by a system, such as a marker coordinate system constructed based on photographic markers has, as the three-dimensional reference coordinate system.

Fig. 2 shows an example of a medical three-dimensional image to be displayed by the display 2 shown in Fig. 1. The medical three-dimensional image shown in Fig. 2 shows a human head. In Fig. 2, reference axes Xo, Yo, and Zo that are directions of the three-dimensional image and coordinate axes of the reference coordinate system are shown together. The reference axes Xo, Yo, and Zo shown in Fig. 2 are set based on landmarks.

The method for setting the reference axes Xo, Yo, and Zo based on landmarks is, for example, the method disclosed in Patent Document 1 (Japanese Published Unexamined Patent Application No. H08-131403) . In Patent Document 1, the reference axes Xo, Yo, and Zo are set according to a rule in which the origin is a midpoint of a line segment connecting left and right earholes, the Xo axis is a straight line parallel to a straight line passing through the left and right eyeball centers, the Yo axis is a straight line perpendicular to a straight line passing through the left and right eyeball centers and the Z axis, the Zo axis is a straight line passing through the left and right eyeball centers and perpendicular to a plane passing through the origin. Accordingly, the reference axis Xo is a straight line penetrating through the human head substantially in the left-right direction, and the reference axis Yo is a straight line penetrating through the human head substantially in the front-back direction, and the reference axis Zo is a straight line penetrating through the human head substantially in the up-down direction.

By setting the Xo axis, the Yo axis, and the Zo axis as reference axes based on landmarks as described above, when evaluating a plurality of medical three-dimensional images displayed according to the reference axes, the images can be directed in almost the same orientation, so that measurements and evaluations can be correctly performed.

When the median of the tooth row is matched with the median of the face, or when the symmetry of the face is diagnosed or treated, setting of the frontal face orientation is important. The frontal face orientation is generally set by specifying landmarks on the facial skin. However, many of landmarks are defined as landmarks for morphological measurement on a radiophotograph, so that when landmarks two-dimensionally defined are three-dimensionally identified, an error occurs, or like a buccal region, etc., it is difficult to reflect a region in which landmarks cannot be set due to its shape in positioning. In addition, the length of a side of a minimum constituent unit (voxel) of CT data is approximately 0.5 mm at minimum, so that it is impossible to specify landmarks with high accuracy in the CT data. Therefore, it is difficult to accurately set the frontal face orientation only by landmark specification.

Therefore, in the present preferred embodiment, the frontal face orientation is adjusted as follows. First, based on medical three-dimensional image data according to the three-dimensional reference coordinate system (Xo, Yo, Zo), a medical three-dimensional image of a human head (hereinafter, referred to as "head image") is displayed together with the reference axes on the display 2, and a reference image to be used as a reference when adjusting the frontal face orientation is displayed on the display 2. A user can rotatively move the whole display image including the head image, the reference image, and the reference axes displayed on the display 2 around screen display axes (screen display axes) Xd, Yd, and Zd set so as to become perpendicular or parallel to the display screen. This is for enabling adjustment of the frontal face orientation based on three-dimensional images viewed from various viewpoint directions.

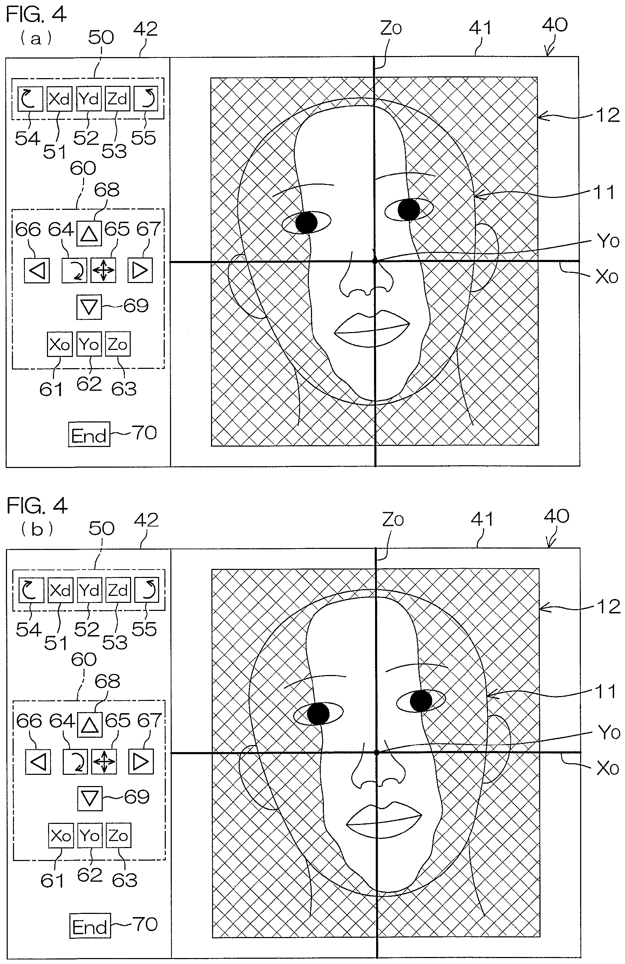

In the present preferred embodiment, the screen display axes Xd, Yd, and Zd are set as follows. The Yd axis is set so as to match a straight line that passes through the center of a display region (hereinafter, referred to as "image display region" indicated by the reference symbol 41 in Fig. 4) for displaying a medical three-dimensional image and extends in the depth direction (direction orthogonal to the screen) of the image display region. The Xd axis is set so as to become parallel to a straight line that passes through the center of the image display region and extends in the transverse direction of the image display region. The Zd axis is set so as to become parallel to a straight line that passes through the center of the image display region and extends in the longitudinal direction of the image display region.

A user adjusts the frontal face orientation by parallel-moving only the medical three-dimensional image with respect to one reference axis specified as an axis to be used as a reference for parallel movement (hereinafter, referred to as "specified reference axis for parallel movement"), and rotatively moving only the medical three-dimensional image around one reference axis specified as a rotation center axis (hereinafter, referred to as "specified reference axis for rotative movement"). When the frontal face orientation is adjusted, the three-dimensional image data on the three-dimensional reference coordinate system is corrected. In other words, the positions of the reference axes Xo, Yo, and Zo with respect to the three-dimensional image are corrected.

Fig. 4 shows a display example of a medical three-dimensional image of a human head on the display 2.

In the display screen region 40 of the display 2, an image display region 41 for displaying a head image (medical three-dimensional image) and an operation region 42 which is disposed on the left side of the image display region 41 and displays various operation buttons are provided. In the operation region 42, an observation direction changing operation portion 50 for changing the observation direction, a display orientation changing operation portion 60 for performing a display orientation change involving parallel movement or rotation with respect to the image reference axes, and an adjustment end button 70, etc., are provided.

The observation direction changing operation portion 50 includes screen display axis specification buttons 51, 52, and 53 for a user to specify a screen display axis around which the whole display image is rotated among the screen display axes Xd, Yd, and Zd, a first observation direction change button 54 for rotating the whole display image by a predetermined amount counterclockwise around the specified screen display axis, and a second observation direction change button 55 for rotating the whole image by a predetermined amount clockwise around the selected screen display axis. The counterclockwise direction and clockwise direction are rotating directions when the specified image display axis is viewed forward. Any one button of the three screen display axis specification buttons 51, 52, and 53 is specified. Therefore, when the first observation direction change button 54 or the second observation direction change button 55 is clicked, the whole display image is rotated around the specified screen display axis in a rotating direction according to the clicked observation direction change button.

The display orientation changing operation portion 60 includes reference axis specification buttons 61, 62, and 63 for a user to specify a reference axis to be used as a reference for parallel movement or rotative movement of the image among the three reference axes Xo, Yo, and Zo, movement mode specification buttons 64 and 65 for a user to specify a rotative movement mode or a parallel movement mode, and four display orientation change buttons 66 to 69. One movement mode specification button 64 is a button for specifying the rotative movement mode, and the other movement mode specification button 65 is a button for specifying the parallel movement mode. Any one of the three reference axis specification buttons 61, 62, and 63 is specified, and either one of the two movement mode selection buttons 64 and 65 is specified.

The four display orientation change buttons 66 to 69 are used for specifying a movement direction when parallel-moving the head image with respect to a specified reference axis for parallel movement in the parallel movement mode. For example, when parallel-moving the head image with respect to the Yo axis, four directions of +Xo, -Xo, +Zo, and -Zo can be specified as a movement direction by the display orientation change buttons 66 to 69. The display orientation change buttons 66 and 67 are also used for rotatively moving the head image around a specified reference axis for rotative movement in the rotative movement mode. When one display orientation change button 66 is operated, the head image is rotated counterclockwise by a predetermined amount around the specified reference axis for rotative movement, and when the other display orientation change button 67 is operated, the head image is rotated clockwise by a predetermined amount around the specified reference axis for rotative movement.

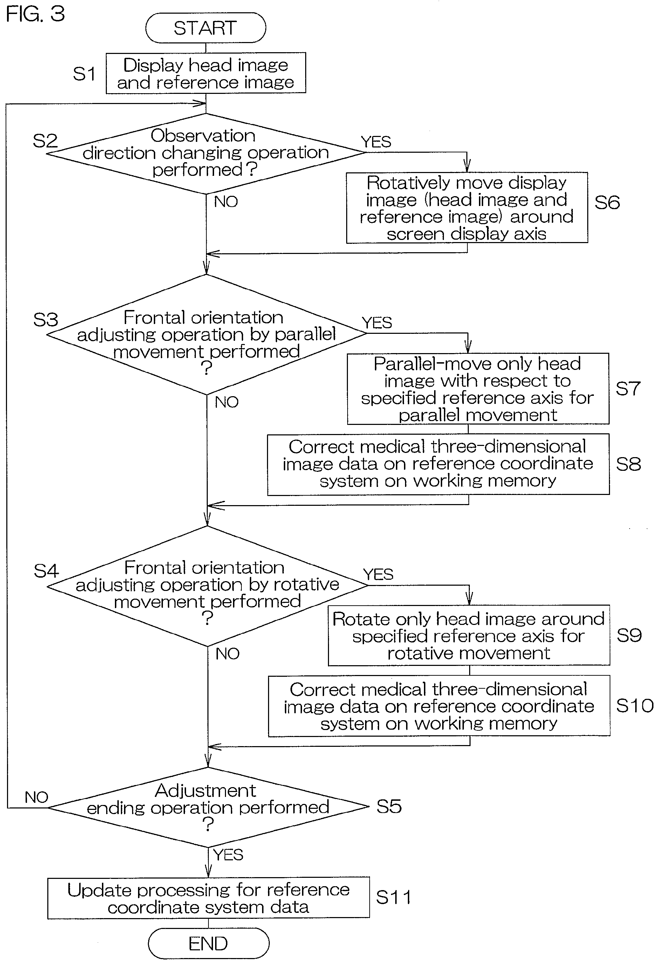

Fig. 3 shows steps of processing for adjusting the frontal face orientation.

For example, as shown in Fig. 4(a), first, the control unit 1 displays a head image 11 to be adjusted, reference axes Xo, Yo, and Zo of the head image 11, and a reference image 12 to be used as a reference for adjustment of the frontal face orientation of the head image 11 in the image display region 41 of the display 2 (Step S1). The head image 11 to be adjusted is displayed based on medical three-dimensional image data selected by a user from, for example, medical three-dimensional image data stored on the hard disk.

To display the head image 11 to be adjusted on the display 2, the control unit 1 reads corresponding medical three-dimensional image data from the hard disk into a predetermined area (hereinafter, referred to as "reference coordinate system data storage area") in a working memory such as a RAM. Next, based on three-dimensional image data on the reference coordinate system Xo, Yo, Zo read into the reference coordinate system data storage area, the control unit 1 converts the three-dimensional image data on the reference coordinate system Xo, Yo, Zo into data on a viewpoint coordinate system Xs, Ys, Zs (not shown). Accordingly, the head image is defined in the viewpoint coordinate systemXs, Ys, Zs. Then, the control unit 1 projects the head image defined in the viewpoint coordinate system onto a predetermined display surface (screen surface). This processing is referred to as "projection processing." Accordingly, three-dimensional image data is converted into two-dimensional image data. The obtained two-dimensional image data is displayed on the display surface.

The viewpoint coordinate system Xs, Ys, Zs is a coordinate system whose origin is a viewpoint, and the Zs axis is set in the line of sight, and the Xs axis and the Ys axis are set in directions orthogonal to the Zs axis. In the viewpoint coordinate system, the above-described screen display axis Yd is parallel to or coaxial with the viewpoint coordinate axis Zs, and the above-described screen display axes Xd and Zd are parallel to the viewpoint coordinate axes Xs and Ys, respectively.

In the example shown in Fig. 4(a), an image in a front view regulated by the reference coordinate system Xo, Yo, Zo of the head image 11 is displayed. Specifically, in the example shown in Fig. 4 (a), the head image 11 is displayed in a posture in which the Yo axis of the reference axes Yo, Xo, and Zo matches the line-of-sight axis Zs of the viewpoint coordinate system, and the reference axes Xo and Zo become parallel to the viewpoint coordinate axes Xs and Ys, respectively. When the head image is displayed in Step S1, the medical three-dimensional image data on the reference coordinate system Xo, Yo, Zo in the reference coordinate system data storage area of the working memory is in an uncorrected state.

The reference image 12 for adjusting the frontal face orientation is a translucent colored plane orthogonal to the reference axis Yo penetrating through the head image 11 substantially in the front-back direction, and intersections of the plane and the face surface of the head image appear as contour lines. In the present preferred embodiment, all reference axes Xo, Yo, and Zo are displayed in the image display region 41.

In this state, a user can perform an operation for rotatively moving the whole display image (the head image 11, the reference image 12, and the reference axes Xo, Yo, and Zo) around the screen display axes Xd, Yd, and Zd to change the observation direction of the display image (hereinafter, referred to as "observation direction changing operation").

In addition, the user can perform an operation for parallel-moving the head image 11 with respect to a reference axis (specified reference axis for parallel movement) specified by the user to adjust the frontal face orientation (hereinafter, referred to as "frontal orientation adjusting operation by parallel movement").

In addition, the user can perform an operation for rotatively moving only the head image 11 around a reference axis (specified reference axis for rotative movement) specified by the user to adjust the frontal face orientation (hereinafter, referred to as "frontal orientation adjusting operation by rotative movement") . Further, the user can perform an adj ustment ending operation for notifying the control unit 1 that the frontal orientation adjusting operation has been ended.

The control unit 1 monitors whether the observation direction changing operation has been performed (Step S2), whether the frontal orientation adjusting operation by parallel movement has been performed (Step S3), whether the frontal orientation adjusting operation by rotative movement has been performed (StepS4), and whether the adjustment ending operation has been performed (Step S5).

The determination whether the observation direction changing operation has been performed of Step S2 is made based on whether the observation direction change button 54 or 55 has been clicked. The determination whether the frontal orientation adjusting operation by parallel movement has been performed of Step S3 is made based on whether any of the display orientation change buttons 66 to 69 has been clicked in the state where the movement mode specification button 65 for specifying the parallel movement mode is specified. The determination whether the frontal orientation adjusting operation by rotative movement of Step S4 is made based on whether the display orientation change button 66 or 67 has been clicked in the state where the movement mode specification button 64 for specifying the rotative movement mode is specified. The determination whether the adjustment ending operation has been performed of Step S5 is made based on whether the adjustment end button 70 has been clicked.

When the observation direction changing operation is performed by the user (YES in Step S2), the control unit 1 rotatively moves the whole display image in a direction according to the observation direction changing operation by a predetermined amount around a screen display axis specified by the display axis specification button 51, 52, or 53 (Step S6). Such rotative movement is achieved by performing the projection processing after rotatively moving the whole three-dimensional figure around the specified screen display axis in the viewpoint coordinate system. Then, the process shifts to Step S3.

When the frontal orientation adjusting operation by parallel movement is performed (YES in Step S3), the control unit 1 parallel-moves only the head image 11 in a direction according to the adjusting operation by a predetermined amount by using the reference axis specified by the reference axis specification button 61, 62, or 63 (specified reference axis for parallel movement) as a reference (Step S7). Such parallel movement is achieved by performing the projection processing after the head image is parallel-moved by a predetermined amount in a specified direction with respect to the specified reference axis for parallel movement in the viewpoint coordinate system.

In this case, the control unit 1 corrects medical three-dimensional image data on the reference coordinate system in the reference coordinate system data storage area of the working memory based on the specified reference axis for parallel movement and specified parallel movement direction and parallel movement amount (Step S8). Accordingly, the medical three-dimensional image data on the reference coordinate system in the reference coordinate system data storage area of the working memory is updated. In this case, the results of correction of the medical three-dimensional image data on the reference coordinate system are equivalent to the results of parallel movement of the specified reference axis for parallel movement by the predetermined amount in a direction opposite to the parallel movement direction of the head image while the head image is fixed.

When the frontal orientation adjusting operation by rotative movement is performed (YES in Step S4), the control unit 1 rotatively moves only the head image 11 in a direction according to the adjusting operation by a predetermined amount around an image reference axis (specified reference axis for rotative movement) specified by the reference axis specification button 61, 62, or 63 (Step S9). Such rotative movement is achieved by performing the projection processing after the head image is rotatively moved in the specified direction by the predetermined amount around the specified reference axis for rotative movement in the viewpoint coordinate system.

In this case, the control unit 1 corrects the medical three-dimensional image data on the reference coordinate system in the reference coordinate system data storage area of the working memory based on the specified reference axis for rotative movement and specified rotative movement direction and rotative movement amount (Step S10). Accordingly, the medical three-dimensional image data on the reference coordinate system in the reference coordinate system data storage area of the working memory is updated. In this case, the results of correction of the medical three-dimensional image data on the reference coordinate system are equivalent to the results of rotative movement of two reference axes other than the specified reference axis for rotative movement in a direction opposite to the rotating direction of the head image by a predetermined amount around the specified reference axis for rotative movement while the head image is fixed.

When an adjustment ending operation is performed (YES in Step S5), the control unit 1 performs processing for updating the reference coordinate system data (Step S11). In detail, the medical three-dimensional image data on the reference coordinate system in the reference coordinate system data storage area of the working memory is substituted for original medical three-dimensional image data in the hard disk. Accordingly, the corresponding medical three-dimensional image data on the reference coordinate system in the hard disk is updated. Then, the present processing is ended.

Parallel movement processing for the head image in Step S7 described above is described in detail. It is assumed that, for example, the image shown in Fig. 4 (a) is displayed in the image display region 41 of the display 2. The user inputs a command for parallel-moving the head image with respect to the Yo axis by referring to the contour lines indicated by the reference image 12 so that the left and right sides (contour lines on both left and right sides) of the face appear symmetrically about the Zo axis. In detail, the user specifies the Yo axis by clicking the reference axis specification button 62, and specifies the parallel movement mode by the movement mode specification button 65. Then, the user clicks any of the display orientation adjustment buttons 66 to 69.

In the head image 11 shown in Fig. 4 (a), the Yo axis is at a position deviated rightward from the center in the left-right direction of the nose as viewed from the front side. To match the center in the left-right direction of the nose with the Yo axis, the head image 11 needs to be parallel-moved rightward (in the +Xo direction) with respect to the Yo axis. Therefore, the user clicks the display orientation adjustment button 67. Then, only the head image 11 is parallel-moved by a predetermined amount rightward (in the +Xo direction) with respect to the Yo axis. When the user clicks the display orientation adjustment button 66, only the head image 11 is parallel-moved by the predetermined amount leftward (in the -Xo direction) with respect to the Yo axis. By repeating this operation, that is, repeating the processes of Step S3, S7, and S8, the user matches the center in the left-right direction of the nose with the Yo axis as shown in Fig. 4(b). In this case, by the process in Step S8, the medical three-dimensional image data on the reference coordinate system in the reference coordinate system data storage area of the working memory is corrected to medical three-dimensional image data corresponding to the positional relationship (the positional relationship shown in Fig. 4 (b)) among the head image 11 after being subjected to frontal orientation adjustment by parallel movement and the reference axes Xo, Yo, and Zo.

The rotative movement processing for the head image 11 of Step S9 is described in detail. First, the case where the frontal face orientation is adjusted by rotating the head image around the Yo axis is described.

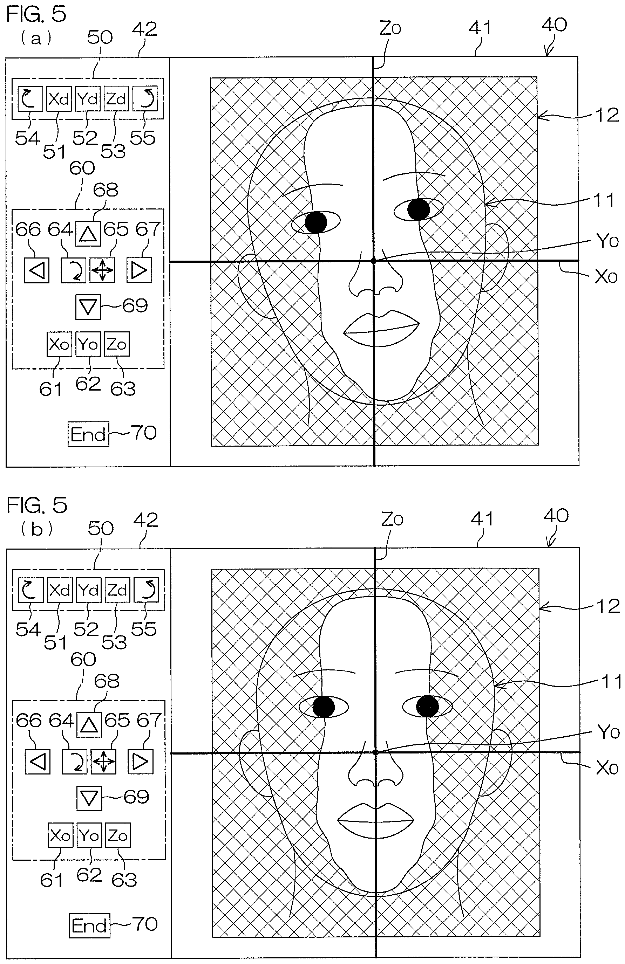

It is assumed that, for example, the image shown in Fig. 5 (a) is displayed in the image display region 41 of the display 2. A user inputs a command for rotating the head image clockwise or counterclockwise around the Yo axis by referring to the contour lines indicated by the reference image 12 so that the left and right sides (contour lines on the left and right sides) of the face appear symmetrically about the Zo axis. In detail, the user specifies the Yo axis by clicking the reference axis specification button 62, and specifies the rotative movement mode by the movement mode specification button 64. Then, the user clicks the display orientation adjustment button 66 or 67.

When the button 66 is clicked, only the head image 11 is rotatively moved by a predetermined amount counterclockwise around the Yo axis. When the button 67 is clicked, only the head image 11 is rotatively moved by a predetermined amount clockwise around the Yo axis. The user repeatedly performs this operation, that is, the processes of Steps S4, S9, and S10 so that the left and right sides (contour lines on the left and right sides) of the face appear symmetrically about the Zo axis as shown in Fig. 5(b). In this case, by the process of Step S10, the medical three-dimensional image data on the reference coordinate system in the reference coordinate system data storage area of the working memory is corrected to medical three-dimensional image data corresponding to the positional relationship among the head image 11 after being subj ected to frontal orientation adjustment by rotative movement and the reference axes Xo, Yo, and Zo (the positional relationship shown in Fig. 5(b)). As a result, the positions of the two reference axes Xo and Zo other than the specified reference axis Yo for rotative movement are corrected with respect to the head image.

The case where the frontal face orientation is adjusted by rotating the head image around the Yo axis after the observation direction is changed is described in detail.

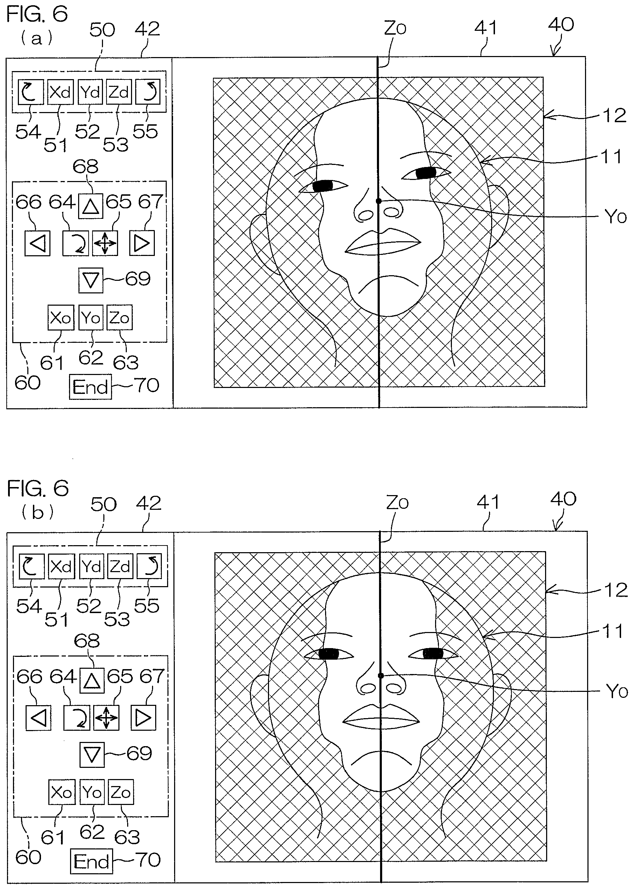

It is assumed that, for example, the image shown in Fig. 5 (a) is displayed in the image display region 41 of the display 2. When a user desires to change the observation direction, the user operates the button in the observation direction changing operation portion 50. For example, it is assumed that the user clicks the first observation direction change button 54 after specifying the screen display axis Xd by clicking the display axis specification button 51. In this case, in Step S6, the whole display image (the head image 11, the reference image 12, and the reference axes Xo, Yo, and Zo) is rotatively moved by a predetermined amount counterclockwise around the screen display axis Xd. Accordingly, the display image becomes, for example, the image shown in Fig. 6(a). In Fig. 6(a), the reference axis Xo is not illustrated. In the present example, the head image 11 is an image as if the face is viewed from an obliquely front position below the face.

The user inputs a command for rotating the head image clockwise or counterclockwise around the Yo axis by referring to the contour lines indicated by the reference image 12 so that the left and right sides (contour lines on the left and right sides) of the face appear symmetrically about the Zo axis. In detail, the user specifies the Yo axis by clicking the reference axis specification button 62, and specifies the rotative movement mode by the movement mode specification button 64. Then, the user clicks the display orientation adjustment button 66 or 67.

When the button 66 is clicked, only the head image 11 is rotatively moved by a predetermined amount counterclockwise around the Yo axis. When the button 67 is clicked, only the head image 11 is rotatively moved by a predetermined amount clockwise around the Yo axis. The user repeatedly performs this operation, that is, the processes of Steps S4, S9, and S10 so that the left and right sides (contour lines on the left and right sides) of the face appear symmetrically about the Zo axis as shown in Fig. 6(b). In Fig. 6(b), the reference axis Xo is not illustrated.

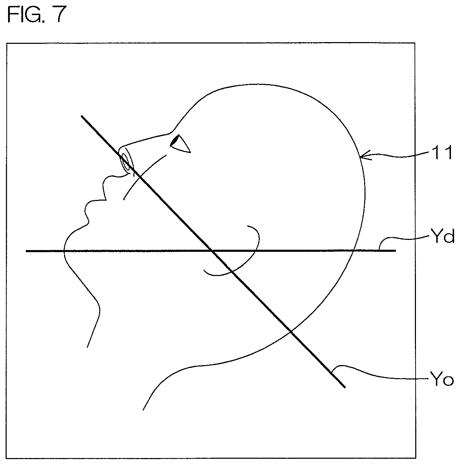

When the observation direction is changed as shown in Fig. 6(a), the specified reference axis Yo for rotative movement becomes not perpendicular to but inclined with respect to the screen as shown in Fig. 7. The reference symbol Yd in Fig. 7 indicates the screen display axis Yd perpendicular to the screen.

Next, the case where the frontal face orientation is adjusted by rotating the head image around the Zo axis is described.

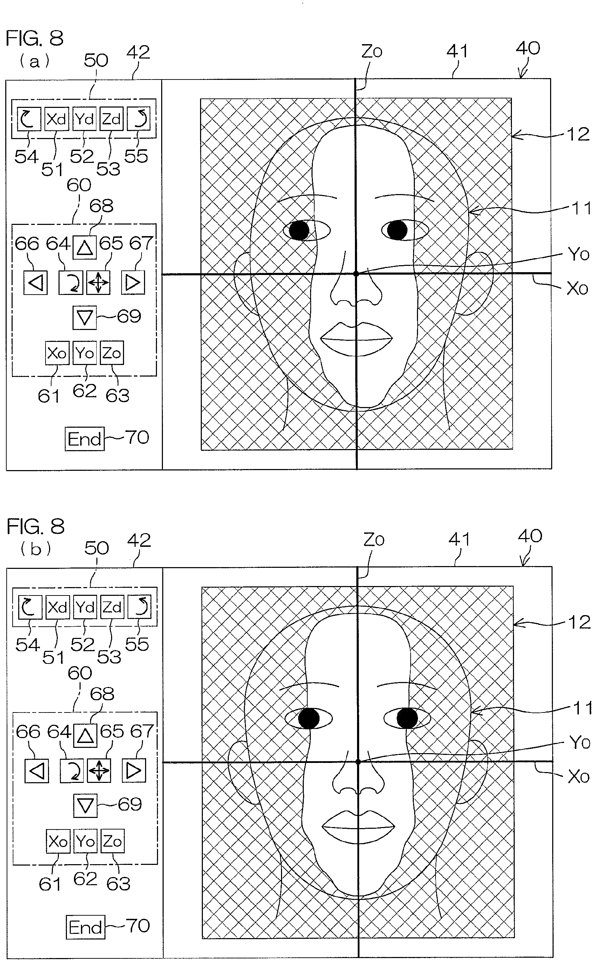

It is assumed that, for example, the image shown in Fig. 8(a) is displayed in the image display region 41 of the display 2. A user inputs a command for rotating the head image clockwise or counterclockwise around the Zo axis by referring to the contour lines indicated by the reference image 12 so that the left and right sides (contour lines on the left and right sides) of the face appear symmetrically about the Zo axis. In detail, the user specifies the Zo axis by clicking the reference axis specification button 63, and specifies the rotative movement mode by the movement mode specification button 64. Then, the user clicks the display orientation adjustment button 66 or 67.

When the button 66 is clicked, only the head image 11 is rotatively moved by a predetermined amount counterclockwise around the Zo axis. When the button 67 is clicked, only the head image 11 is rotatively moved by a predetermined amount clockwise around the Zo axis. The user repeatedly performs this operation, that is, the processes of Steps S4, S9, and S10 so that the left and right sides (contour lines on the left and right sides) of the face appear symmetrically about the Zo axis as shown in Fig. 8 (b) . In this case, by the process of Step S10, the medical three-dimensional image data on the reference coordinate system in the reference coordinate system data storage area of the working memory is corrected to medical three-dimensional image data corresponding to the positional relationship (the positional relationship shown in Fig. 8 (b)) among the head image 11 after being subjected to frontal orientation adjustment by rotative movement and the reference axes Xo, Yo, and Zo. As a result, the two reference axes Xo and Yo other than the specified reference axis Zo for rotative movement are corrected with respect to the head image.

The case where the frontal face orientation is adjusted by rotating the head image around the Zo axis after the observation direction is changed is described in detail.

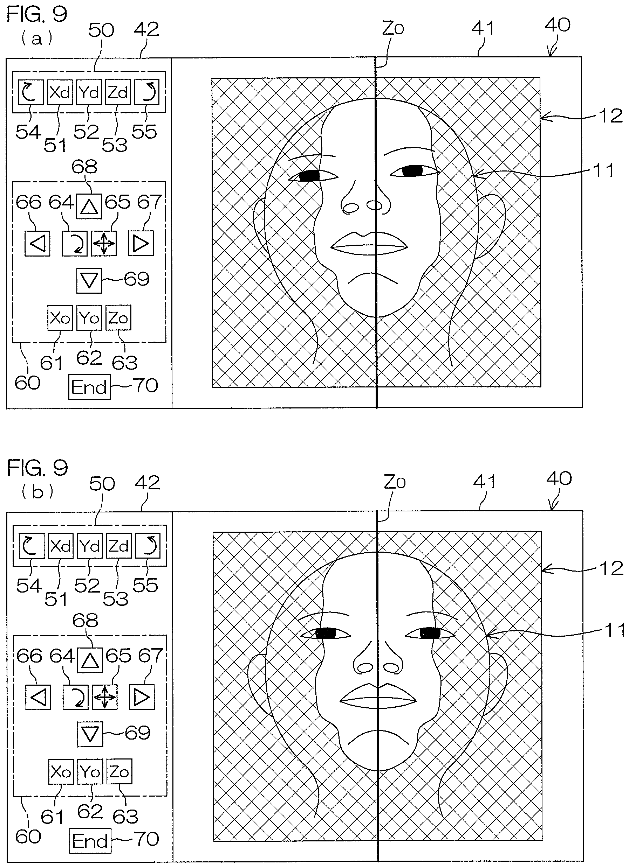

It is assumed that, for example, the image shown in Fig. 8 (a) is displayed in the image display region 41 of the display 2. When a user desires to change the observation direction, the user operates a button in the observation direction changing operation portion 50. For example, it is assumed that the user clicks the first observation direction change button 54 after specifying the screen display axis Xd by clicking the display axis specification button 51. In this case, in Step S6, the whole display image is rotatively moved by a predetermined amount counterclockwise around the screen display axis Xd. Accordingly, the display image becomes, for example, the image shown in Fig. 9(a). In Fig. 9(a), the reference axes Xo and Yo are not illustrated. In the present example, the head image 11 is an image as if the face is viewed from an obliquely front position below the face.

The user inputs a command for rotating the head image clockwise or counterclockwise around the Zo axis by referring to the contour lines indicated by the reference image 12 so that the left and right sides (contour lines on the left and right sides) appear symmetrically about the Zo axis. In detail, the user specifies the Zo axis by clicking the reference axis specification button 63, and specifies the rotative movement mode by the movement mode specification button 64. Then, the user clicks the display orientation adjustment button 66 or 67.

When the button 66 is clicked, only the head image 11 is rotatively moved by a predetermined amount counterclockwise around the Zo axis. When the button 67 is clicked, only the head image 11 is rotatively moved by a predetermined amount clockwise around the Zo axis. The user repeatedly performs this operation, that is, the processes of Steps S4, S9, and S10 so that the left and right sides (contour lines on the left and right sides) of the face appear symmetrically about the Zo axis as shown in Fig. 9(b). In Fig. 9(b), the reference axes Xo and Yo are not illustrated.

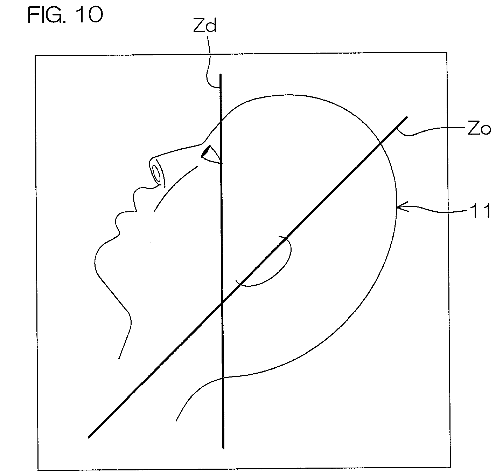

When the observation direction is changed as shown in Fig. 9(a), the specified reference axis Zo for rotative movement becomes not parallel to but inclined with respect to the screen. The reference symbol Zd in Fig. 10 indicates the screen display axis Zd parallel to the screen.

The user can rotate only the head image 11 around the reference axis Xo although this is not illustrated. In detail, the user specifies the Xo axis by clicking the reference axis specification button 61, and specifies the rotative movement mode by the movement mode specification button 64. Then, the user clicks the display orientation adjustment button 66 or 67. When the head image 11 is rotated around the reference axis Xo, the positions of the two reference axes Yo and Zo other than the specified reference axis Xo for rotative movement are corrected with respect to the head image.

According to the preferred embodiment described above, when adjusting the frontal face orientation of the head image (medical three-dimensional image) 11, the reference image 12 such as the plane orthogonal to the reference axis (Yo axis) extending in the front-back direction of the face is displayed, so that the frontal face orientation of the head image 11 can be easily adjusted.

According to the preferred embodiment described above, the frontal face orientation of the head image 11 can be adjusted from a plurality of observation directions, so that the frontal face orientation can be more accurately adjusted. Specifically, not only can the limited orientations such as the front face, upper face, and lower face, the head image and the reference image be compared from obliquely below or obliquely above by changing the observation direction, the head image can be accurately positioned by referring to all forms of the maxillofacial morphology with irregularities.

Orientations orthogonal to the screen axes, such as the front face, the left side face, the right side face, the upper face, and the lower face, are basic observation orientations, however, a stereoscopic image has irregularities, and depending on the viewpoint direction, necessary portions are hidden and cannot be used for positioning. However, in the preferred embodiment described above, the observation orientation can be freely moved together with the head image and the reference image, so that the problem can be solved.

The data to be adjusted is three-dimensional image data, however, the image to be displayed on the screen is a two-dimensional image, so that the orientations in which the left and right sides are simultaneously displayed are only the front face, the upper face, and the lower face. The regions that can be referred to for positioning the head image in the three orientations are limited. Therefore, in the preferred embodiment described above, by freely rotating the head image 360 degrees, the head image and the reference image can be observed in a plurality of viewpoint directions, so that truly stereoscopic positioning is possible. Based on changes of the contour lines or a moire pattern projected in the third image reference axis direction when only the head image is parallel-moved or rotatively moved, the head image can be positioned so that the head image becomes most bilaterally symmetrical by referring to the position and orientation that make the contour lines or moire pattern most symmetrical.

Further, according to the preferred embodiment described above, based on the results of parallel movement of the head image 11 with respect to a specified reference axis for parallel movement and the results of rotative movement of the head image 11 around a specified reference axis for rotative movement, two reference axes other than the specified rotation center axis are corrected, so that it is not necessary to specify landmarks that are hard to accurately specify. Landmark specification is unnecessary, so that the problems such as the resolution of the head image, errors when specifying landmarks, and unavailability of regions in which landmarks cannot be defined for positioning, can be solved.

A preferred embodiment of the present invention is described above, however, the present invention can be carried out with other preferred embodiments. For example, a means for rotatively moving the whole display image around the screen display axis for changing the observation direction is provided, however, instead of or in addition to the means, a means for parallel-moving the whole display image with respect to the screen display axis may be provided.

As a reference image, in addition to a reference plane orthogonal to a predetermined image reference axis (hereinafter, referred to as a third image reference axis, that is Yo axis when adjusting the frontal face orientation) set or specified for the reference image, cells and face symmetric model outline orthogonal to the third image reference axis, and contour lines and moire projected in the third image reference axis direction, etc., can be used.

In addition, the reference image such as the plane or cells, orthogonal to the third image reference axis is preferably parallel-moved with respect to the third image reference axis. The transparency and color of the reference image are preferably changeable. Accordingly, the position and display pattern of the reference image can be adjusted so that the positional relationship between the reference image and the head image can be easily grasped. After adjusting the position and display pattern of the reference image, by referring to the shape of a region in which the reference plane and the head image overlap each other, the cells, and the bilateral symmetrical model outline, the position at which the head image becomes most bilaterally symmetrical can be identified.

It is also possible that a face photograph of a patient or a face feature outline extracted from the face photograph is displayed as a reference image on a plane orthogonal to the third image reference axis. Accordingly, the head image can be positioned in a face orientation adopted when the face photograph is photographed. Similarly, it is also possible that based on a CT image photographed and positioned in the past, an image in an orientation orthogonal to the third image reference axis is displayed on the plane. Accordingly, the head image can be positioned in the display orientation evaluated in the past, so that a change from the past can be quantitatively evaluated.

In addition, it is also possible that, in addition to the frontal face photograph and the left and right side photographs, a plurality of photographs taken from a plurality of directions at angles of 10 degrees, 20 degrees, and 30 degrees right oblique to the front and angles of 10 degrees, 20 degrees, and 30 degrees left oblique to the front, and the photographing directions are registered in advance, and when a head image observation direction is specified, a photograph in the orientation corresponding to the observation direction is displayed as a reference image on a plane orthogonal to the viewpoint direction. Accordingly, not only can photographs be taken from the front and lateral directions but also photographs taken from oblique directions can be positioned while confirming the positional relationship with the reference image, so that more stereoscopic orientation adjustments can be made. In this case, the transparency and color of the reference image are also preferably changeable. It is also possible that image processing such as outline extraction from a photograph image as a reference is performed and the extracted outline is displayed as a reference image.

Further, instead of the photographs, three-dimensional information such as CT image data, MRI data, and three-dimensional camera photographic data taken in the past can also be utilized. In this case, from the three-dimensional information registered in advance as a reference material, an image in a direction matching the head image observation direction is created as a two-dimensional image, and the two-dimensional image can be displayed as a reference image on a plane orthogonal to the head image observation direction. This method is effective when a CT image, etc., of the same patient positioned for diagnostic evaluation in the past is available, and when a reference material including stereoscopic shape information such as a three-dimensional camera image is available.

It is preferable that a plane orthogonal to the third image reference axis or a plane orthogonal to the observation direction on which the reference image is displayed can be parallel-moved along the third image reference axis or the observation direction, and the color and transparency of the reference material such as a CT image or a three-dimensional photographic image displayed on the plane are adjustable. It is also possible that the outline of the reference material is extracted and displayed on the plane. Accordingly, the positional relationship between the head image and the reference image can be easily grasped. Even when the reference material is three-dimensional information, an image to be displayed as a reference image is a two-dimensional image, so that the operations such as the adjustments of the color and transparency of the reference image and outline extraction are easy, and the process time is short.

When three-dimensional images are directly overlaid, the inner region of the overlaid portion becomes invisible (the forward region is displayed, and an image behind the forward region is hidden, so that it is hard to grasp the overlap in the depth direction), and it is hard to grasp whether the positions in the depth direction of the two images match exactly or loosely, so that accurate positioning is difficult. In the method described above, according to specification of an observation direction, a reference image corresponding to the viewpoint direction is created, and the reference image is displayed as a two-dimensional image on a plane orthogonal to the observation direction. Thus, a head image that is a three-dimensional image and a reference image that is a two-dimensional image are overlaid in a plurality of directions, so that the problem that is posed when overlaying three-dimensional images is solved, and stereoscopic accurate positioning is realized.

Depending on setting of the frontal face orientation of the head image, the symmetry evaluation changes, so that the setting of the orientation is an important matter that determines the results of treatment. Therefore, correctness corresponding to various definitions of the frontal orientation and repeatedly reproducible accuracy are important. Therefore, when a patient determines his/her desired frontal orientation in front of a mirror and desires realization of symmetry in the determined orientation, a three-dimensional camera photograph is photographed in the determined orientation, and data thereof may be used as a reference material for creating a positioning reference image. Accordingly, a head image can be accurately positioned in an orientation matching the three-dimensional camera photograph taken in the same orientation. In this case, not only are images in the front face, the side face, and the upper and lower face orientations are displayed on a plane parallel to the screen, but also a reference image can be created in other viewpoint directions because the reference image has stereoscopic information. Therefore, according to determination of an observation direction, a two-dimensional face photograph (reference image) in the corresponding viewpoint direction can be created and displayed on a plane orthogonal to the observation direction. Accordingly, when photographing a three-dimensional image such as a CT image, a difficult frontal orientation that a patient desires can be positioned by another means, for example, a three-dimensional material positioned on a patient's actual body.

Various other design changes can be made within the scope of the matters described in claims.

### Description of the Reference Symbols

- 1: Control unit
- 2: Display
- 3: Keyboard
- 4: Mouse
- 5: Storage medium
- 11: Head image
- 12: Reference image
- 50: Observation direction changing operation portion
- 60: Display orientation changing operation portion

## Claims

1. A display orientation adjustment device for a medical three-dimensional image, comprising:
a means for making a display means (2) display a medical three-dimensional image (11) based on medical three-dimensional image data according to a predetermined three-dimensional reference coordinate system (X₀, Y₀, Z₀)
a means for making the display means (2) display a reference image (12) to be used as a reference for adjustment of a display orientation of the medical three-dimensional image (11);
an observation direction change command input means (50) for inputting an observation direction change command for changing an observation direction of a display image displayed by the display means (2);
an observation direction changing means for moving the whole display image including the medical three-dimensional image (11) and the reference image (12) according to an observation direction change command input by the observation direction change command input means;
a display orientation change command input means (60) for inputting a command for rotatively moving only the medical three-dimensional image (11) of the medical three-dimensional image (11) and the reference image (12) displayed by the display means (2) around a first image reference axis specified among image reference axes of the three-dimensional reference coordinate system (X₀, Y₀, Z₀);
a display orientation changing means for rotatively moving only the medical three-dimensional image (11) around the first image reference axis according to a display orientation change command input by the display orientation change command input means (60);
and
a first correcting means for correcting medical three-dimensional image data on the three-dimensional reference coordinate system (X₀, Y₀, Z₀) according to the rotative movement of the medical three-dimensional image by the display orientation changing means.

2. The display orientation adjustment device for a medical three-dimensional image according to Claim 1, wherein the first correcting means corrects two image reference axes other than the first image reference axis based on the first image reference axis and a direction and a magnitude of the rotative movement of the medical three-dimensional image (11) by the display orientation changing means.

3. The display orientation adjustment device for a medical three-dimensional image according to Claim 1 or 2, further comprising:
a parallel movement command input means for inputting a parallel movement command for parallel-moving the medical three-dimensional image (11) displayed by the display means (2) relative to a second image reference axis specified among the three-dimensional image reference axes;
a parallel moving means for parallel-moving the medical three-dimensional image (11) displayed by the display means (2) relative to the second image reference axis according to a parallel movement command input by the parallel movement command input means; and
a second correcting means for correcting medical three-dimensional image data on the three-dimensional reference coordinate system (X₀, Y₀, Z₀) according to parallel movement by the parallel moving means.

4. A computer readable medium comprising a display orientation adjustment program for a medical three-dimensional image, for making a computer function as: a means for making a display means (2) display a medical three-dimensional image (11) based on medical three-dimensional image data according to a predetermined three-dimensional reference coordinate system(X₀, Y₀, Z₀); a means for making the display means display a reference image (12) to be used as a reference for adjustment of a display orientation of the medical three-dimensional image (11); an observation direction changing means for moving the whole display image including the medical three-dimensional image (11) and the reference image (12) according to a given observation direction change command; a display orientation changing means for rotatively moving only the medical three-dimensional image (11) of the medical three-dimensional image (11) and the reference image (12) displayed by the display means (2) around a first image reference axis specified among image reference axes of the three-dimensional reference coordinate system (X₀, Y₀, Z₀) according to a given display orientation change command; and a first correcting means for correcting medical three-dimensional image data on the three-dimensional reference coordinate system according to the rotative movement of the medical three-dimensional image (11) by the display orientation changing means.

5. The computer readable medium according to Claim 4, wherein the first correcting means corrects two image reference axes other than the first image reference axis based on the first image reference axis and a direction and a magnitude of the rotative movement of the medical three-dimensional image (11) by the display orientation changing means.

6. The computer readable medium according to Claim 4 or 5, further comprising a program for making a computer function as: a parallel moving means for parallel-moving the medical three-dimensional image (11) displayed by the display means (2) relative to a second image reference axis specified among the three-dimensional image reference axes according to a given parallel movement command; and a means for correcting medical three-dimensional image data on the three-dimensional reference coordinate system(X₀, Y₀, Z₀) according to the parallel movement by the parallel moving means.

## Patentansprüche

1. Vorrichtung zur Einstellung der Orientierung einer Anzeige, und zwar für ein medizinisches dreidimensionales Bild, wobei die Vorrichtung aufweist:
ein Mittel, um ein Anzeigemittel (2) dazu zu bringen, ein medizinisches dreidimensionales Bild (11) auf der Grundlage von medizinischen dreidimensionalen Bilddaten gemäß einem vorbestimmten dreidimensionalen Referenz-Koordinatensystem (X₀, Y₀, Z₀) anzuzeigen;
ein Mittel, um das Anzeigemittel (2) dazu zu bringen, ein Referenz-Bild (12) anzuzeigen, das als eine Referenz zur Einstellung einer Orientierung der Anzeige des medizinischen dreidimensionalen Bildes (11) zu verwenden ist;
ein Betrachtungsrichtungsveränderungs-Befehls-Eingabemittel (50) zum Eingeben eines Betrachtungsrichtungsveränderungs-Befehls, und zwar zum Verändern einer Betrachtungsrichtung eines Anzeigebildes, das von dem Anzeigemittel (2) angezeigt wird;
einem Betrachtungsrichtungs-Veränderungsmittel zum Bewegen des gesamten Anzeigebildes, das das medizinische dreidimensionale Bild (11) und das Referenz-Bild (12) beinhaltet, und zwar gemäß einer Betrachtungsrichtungsveränderungs-Befehls-Eingabe mittels des Betrachtungsrichtungsveränderungs-Befehls-Eingabemittels;
einem Anzeigeorientierungsveränderungs-Befehls-Eingabemittel (60) zum Eingeben eines Befehls zum drehenden Bewegen von nur dem medizinischen dreidimensionalen Bild (11) von dem medizinischen dreidimensionalen Bild (11) und dem Referenz-Bild (12), die von dem Anzeigemittel (2) angezeigt werden, und zwar um eine erste Bildreferenzachse herum, die aus Bildreferenzachsen des dreidimensionalen Referenz-Koordinatensystems (X₀, Y₀, Z₀) spezifiziert ist;
ein Anzeigeorientierungs-Veränderungsmittel zum drehenden Bewegen von nur dem medizinischen dreidimensionalen Bild (11) um die erste Bildreferenzachse herum, und zwar gemäß einer Anzeigeorientierungsveränderungs-Befehls-Eingabe mittels des Anzeigeorientierungsveränderungs-Befehls-Eingabemittels (60);
und
ein erstes Korrekturmittel zum Korrigieren von medizinischen dreidimensionalen Bilddaten an dem dreidimensionalen Referenz-Koordinatensystem (X₀, Y₀, Z₀), und zwar gemäß der drehenden Bewegung des medizinischen dreidimensionalen Bildes mittels des Anzeigeorientierungs-Änderungsmittels.

2. Vorrichtung zur Einstellung der Orientierung einer Anzeige, und zwar für ein medizinisches dreidimensionales Bild, gemäß Anspruch 1, wobei das erste Korrekturmittel zwei Bildreferenzachsen korrigiert, die sich von der ersten Bildreferenzachse unterscheiden, und zwar auf der Grundlage der ersten Bildreferenzachse und einer Richtung und einer Größe der Drehbewegung des medizinischen dreidimensionalen Bildes (11) mittels des Anzeigeorientierungs-Veränderungsmittels.

3. Vorrichtung zur Einstellung der Orientierung einer Anzeige, und zwar für ein medizinisches dreidimensionales Bild, gemäß Anspruch 1 oder 2, ferner mit:
einem Parallelbewegungs-Befehls-Eingabemittel zum Eingeben eines Parallelbewegungs-Befehls zum parallelen Bewegen des medizinischen dreidimensionalen Bildes (11), das von dem Anzeigemittel (2) angezeigt wird, und zwar relativ zu einer zweiten Bildreferenzachse, die aus den dreidimensionalen Bildreferenzachsen spezifiziert ist;
ein Parallelbewegungs-Mittel zum parallelen Bewegen des medizinischen dreidimensionalen Bildes (11), das von dem Anzeigemittel (2) angezeigt wird, relativ zu der zweiten Bildreferenzachse, und zwar gemäß einer Parallelbewegungs-Befehls-Eingabe mittels des Parallelbewegungs-Befehls-Eingabemittels; und
einem zweiten Korrekturmittel zum Korrigieren von medizinischen dreidimensionalen Bilddaten an dem dreidimensionalen Referenz-Koordinatensystem (X₀, Y₀, Z₀), und zwar gemäß der Parallelbewegung mittels des Parallelbewegungs-Mittels.

4. Computerlesbares Medium mit einem Programm zur Einstellung der Orientierung einer Anzeige, und zwar für ein medizinisches dreidimensionales Bild, um einen Computer arbeiten zu lassen als: ein Mittel, um ein Anzeigemittel (2) dazu zu bringen, ein medizinisches dreidimensionales Bild (11) auf der Grundlage von medizinischen dreidimensionalen Bilddaten gemäß einem vorbestimmten dreidimensionalen Referenz-Koordinatensystem (X₀, Y₀, Z₀) anzuzeigen; ein Mittel, um das Anzeigemittel dazu zu bringen, ein Referenz-Bild (12) anzuzeigen, das als eine Referenz zur Einstellung einer Orientierung der Anzeige des medizinischen dreidimensionalen Bildes (11) zu verwenden ist; ein Betrachtungsrichtungs-Veränderungsmittel zum Bewegen des gesamten Anzeigebildes einschließlich des medizinischen dreidimensionalen Bildes (11) und des Referenz-Bildes (12) gemäß einem gegebenen Betrachtungsrichtungsveränderungs-Befehl; ein Anzeigeorientierungs-Veränderungsmittel zum drehenden Bewegen von nur dem medizinischen dreidimensionalen Bild (11) aus dem medizinischen dreidimensionalen Bild (11) und dem Referenz-Bild (12), die von dem Anzeigemittel (2) angezeigt werden, und zwar um eine erste Bildreferenzachse herum, die aus Bildreferenzachsen des dreidimensionalen Referenz-Koordinatensystems (X₀, Y₀, Z₀) gemäß einem gegebenen Anzeigeorientierungsveränderungs-Befehls spezifiziert ist; und ein erstes Korrekturmittel zum Korrigieren der medizinischen dreidimensionalen Bilddaten an dem dreidimensionalen Referenz-Koordinatensystem gemäß der drehenden Bewegung des medizinischen dreidimensionalen Bildes (11) mittels des Anzeigeorientierungs-Veränderungsmittels.

5. Computerlesbares Medium nach Anspruch 4, wobei das erste Korrekturmittel zwei Bildreferenzachsen korrigiert, die sich von der Bildreferenzachse unterscheiden, und zwar auf der Grundlage der ersten Bildreferenzachse und einer Richtung und einer Größe der drehenden Bewegung des medizinischen dreidimensionalen Bildes (11) mittels des Anzeigeorientierungs-Veränderungsmittels.

6. Computerlesbares Medium nach Anspruch 4 oder 5, ferner mit einem Programm, um einen Computer funktionieren zu lassen als: ein Parallelbewegungs-Mittel zum parallelen Bewegen des medizinischen dreidimensionalen Bildes (11), das von dem Anzeigemittel (2) angezeigt wird, und zwar relativ zu einer zweiten Bildreferenzachse, die aus den dreidimensionalen Bildreferenzachsen gemäß einem gegebenen Parallelbewegungs-Befehls spezifiziert ist; und ein Mittel zum Korrigieren der medizinischen dreidimensionalen Bilddaten an dem dreidimensionalen Referenz-Koordinatensystem (X₀, Y₀, Z₀) gemäß der parallelen Bewegung mittels des Parallelbewegungs-Mittels.

## Revendications

1. Dispositif de réglage d'orientation d'affichage pour image médicale tridimensionnelle, comprenant :
un moyen amenant un moyen d'affichage (2) à afficher une image médicale tridimensionnelle (11) sur la base de données d'image médicale tridimensionnelle selon un système de coordonnées de référence tridimensionnel prédéterminé (X₀, Y₀, Z₀) ;
un moyen amenant le moyen d'affichage (2) à afficher une image de référence (12) destinée à être utilisée comme référence pour le réglage d'une orientation d'affichage de l'image médicale tridimensionnelle (11) ;
un moyen d'entrée de commande de changement de sens d'observation (50) permettant d'entrer une commande de changement de sens d'observation pour changer un sens d'observation d'une image d'affichage affichée par le moyen d'affichage (2) ;
un moyen de changement de sens d'observation permettant de déplacer l'image d'affichage entière, dont l'image médicale tridimensionnelle (11) et l'image de référence (12), selon une commande de changement de sens d'observation entrée par le moyen d'entrée de commande de changement de sens d'observation ;
un moyen d'entrée de commande de changement d'orientation d'affichage (60) permettant d'entrer une commande pour déplacer en rotation uniquement l'image médicale tridimensionnelle (11) de l'image médicale tridimensionnelle (11) et de l'image de référence (12) affichées par le moyen d'affichage (2) autour d'un premier axe de référence d'image spécifié parmi des axes de référence d'image du système de coordonnées de référence tridimensionnel (X₀, Y₀, Z₀) ;
un moyen de changement d'orientation d'affichage permettant de déplacer en rotation uniquement l'image médicale tridimensionnelle (11) autour du premier axe de référence d'image selon une commande de changement d'orientation d'affichage entrée par le moyen d'entrée de commande de changement d'orientation d'affichage (60) ; et
un premier moyen de correction permettant de corriger les données d'image médicale tridimensionnelle sur le système de coordonnées de référence tridimensionnel (X₀, Y₀, Z₀) selon le déplacement de rotation de l'image médicale tridimensionnelle par le moyen de changement d'orientation d'affichage.

2. Dispositif de réglage d'orientation d'affichage pour image médicale tridimensionnelle selon la revendication 1, dans lequel le premier moyen de correction corrige deux axes de référence d'image différents du premier axe de référence d'image sur la base du premier axe de référence d'image et d'un sens et d'une importance du déplacement relatif de l'image médicale tridimensionnelle (11) par le moyen de changement d'orientation d'affichage.

3. Dispositif de réglage d'orientation d'affichage pour image médicale tridimensionnelle selon la revendication 1 ou 2, comprenant en outre :
un moyen d'entrée de commande de déplacement parallèle permettant d'entrer une commande de déplacement parallèle pour déplacer parallèlement l'image médicale tridimensionnelle (11) affichée par le moyen d'affichage (2) par rapport à un second axe de référence d'image spécifié parmi les axes de référence d'image tridimensionnelle ;
un moyen de déplacement parallèle permettant de déplacer parallèlement l'image médicale tridimensionnelle (11) affichée par le moyen d'affichage (2) par rapport au second axe de référence d'image selon une commande de déplacement parallèle entrée par le moyen d'entrée de commande de déplacement parallèle ; et
un second moyen de correction permettant de corriger les données d'image médicale tridimensionnelle sur le système de coordonnées de référence tridimensionnel (X₀, Y₀, Z₀) selon un déplacement parallèle par le moyen de déplacement parallèle.

4. Support lisible par ordinateur comprenant un programme de réglage d'orientation d'affichage pour image médicale tridimensionnelle, amenant une fonction informatique à fonctionner comme : un moyen amenant un moyen d'affichage (2) à afficher une image médicale tridimensionnelle (11) sur la base de données d'image médicale tridimensionnelle selon un système de coordonnées de référence tridimensionnel prédéterminé (X₀, Y₀, Z₀) ; un moyen amenant le moyen d'affichage à afficher une image de référence (12) destinée à être utilisée comme référence pour le réglage d'une orientation d'affichage de l'image médicale tridimensionnelle (11) ; un moyen de changement de sens d'observation permettant de déplacer l'image d'affichage entière, dont l'image médicale tridimensionnelle (11) et l'image de référence (12), selon une commande de changement de sens d'observation donnée ; un moyen de changement d'orientation d'affichage permettant de déplacer en rotation uniquement l'image médicale tridimensionnelle (11) de l'image médicale tridimensionnelle (11) et de l'image de référence (12) affichées par le moyen d'affichage (2) autour d'un premier axe de référence d'image spécifié parmi des axes de référence d'image du système de coordonnées de référence tridimensionnel (X₀, Y₀, Z₀) selon une commande de changement d'orientation d'affichage ; et un premier moyen de correction permettant de corriger les données d'image médicale tridimensionnelle sur le système de coordonnées de référence tridimensionnel selon le déplacement de rotation de l'image médicale tridimensionnelle (11) par le moyen de changement d'orientation d'affichage.

5. Support lisible par ordinateur selon la revendication 4, dans lequel le premier moyen de correction corrige deux axes de référence d'image différents du premier axe de référence d'image sur la base du premier axe de référence d'image et d'un sens et d'une importance du déplacement relatif de l'image médicale tridimensionnelle (11) par le moyen de changement d'orientation d'affichage.

6. Support lisible par ordinateur selon la revendication 4 ou 5, comprenant en outre un programme amenant une fonction informatique à fonctionner comme : un moyen de déplacement parallèle permettant de déplacer parallèlement l'image médicale tridimensionnelle (11) affichée par le moyen d'affichage (2) par rapport à un second axe de référence d'image spécifié parmi les axes de référence d'image tridimensionnelle selon une commande de déplacement parallèle donnée ; et un moyen permettant de corriger les données d'image médicale tridimensionnelle sur le système de coordonnées de référence tridimensionnel (X₀, Y₀, Z₀) selon le déplacement parallèle par le moyen de déplacement parallèle.
